# EUROPEAN PATENT SPECIFICATION

(11) **EP 3 578 189 B1**
(45) Date of publication and mention of the grant of the patent: **31.08.2022**
(21) Application number: 17895438.4
(22) Date of filing: 03.02.2017
(51) Int. Cl.: A61K 38/39, A61P 31/04, A61K 33/34, A61K 33/38, A61P 17/02, A61K 33/24, A61L 27/24, A61L 27/54, A61L 27/56, A61L 27/58, A61K 35/50

(54) **COLLAGEN MEMBRANE OR MATRIX WITH ANTIMICROBIAL PROPERTIES**
KOLLAGENMEMBRAN ODER MATRIX MIT ANTIMIKROBIELLEN EIGENSCHAFTEN
MATRICE OU MEMBRANE DE COLLAGÈNE À PROPRIÉTÉS ANTIMICROBIENNES

(43) Date of publication of application: 11.12.2019
(73) Proprietor: Biocellix SpA, Las Condes, Santiago (CL)
(72) Inventor: DIAZ MORALES, Maria Ines, Ñuñoa, Santiago (CL)
(74) Representative: Curell Suñol S.L.P.
(86) International application number: PCT/CL2017/050005
(87) International publication number: WO 2018/141075

(56) References cited:
- WO-A1-2010/033860
- WO-A1-2014/191955
- WO-A1-2016/168752
- WO-A2-2006/135843
- WO-A2-2008/057162
- US-A1- 2004 048 796
- US-A1- 2012 189 586
- ALBU MADALINA G ET AL: "Preparation and Biological Activity of New Collagen Composites, Part I: Collagen/Zinc Titanate Nanocomposites", APPLIED BIOCHEMISTRY AND BIOTECHNOLOGY, HUMANA PRESS INC, NEW YORK, vol. 180, no. 1, 2 May 2016 (2016-05-02), pages 177-193, XP036047368, ISSN: 0273-2289, DOI: 10.1007/S12010-016-2092-X [retrieved on 2016-05-02]
- BAHAREH SALEHI ET AL: "Investigation of antibacterial effect of Cadmium Oxide nanoparticles on Staphylococcus Aureus bacteria", JOURNAL OF NANOBIOTECHNOLOGY, vol. 12, no. 1, 1 December 2014 (2014-12-01), XP055708664, DOI: 10.1186/s12951-014-0026-8
- Shekhar Agnihotri ET AL: "Development of Nano-Antimicrobial Biomaterials for Biomedical Applications" In: "Carbon and Oxide Nanostructures", 1 January 2017 (2017-01-01), Springer Berlin Heidelberg, Berlin, Heidelberg, XP055708774, ISSN: 1869-8433 ISBN: 978-3-642-14673-2 vol. 66, pages 479-545, DOI: 10.1007/978-981-10-3328-5_12, * the whole document * * table 2 *
- MADALINA GRIGORE ET AL: "Collagen-Nanoparticles Composites for Wound Healing and Infection Control", METALS, vol. 7, no. 12, 23 November 2017 (2017-11-23), page 516, XP055708481, DOI: 10.3390/met7120516
- OYARZUN-AMPUERO, F. A. et al.: "Nanoparticles for the Treatment of Wounds", Current pharmaceutical design, vol. 21, August 2015 (2015-08), pages 4329-4341, XP055533659,
- SINGH, R. et al.: "Chitin membranes containing silver nanoparticles for wound dressing application", International wound journal, vol. 11, no. 3, 2014, pages 264-268, XP055533665,
- KESTING, M. R. et al.: "The role of allogenic amniotic membrane in burn treatment", Journal of burn care & research, vol. 29, no. 6, 2008, pages 907-916, XP055624886,
- HABERAL, M. et al.: "The use of silver nitrate-incorporated amniotic membrane as a temporary dressing", Burns, vol. 13, no. 2, April 1987 (1987-04), pages 159-163, XP055533674,
- ILIC, D. et al.: "Human amniotic membrane grafts in therapy of chronic non-healing wounds", British Medical Bulletin, vol. 117, no. 1, January 2016 (2016-01), pages 59-67, XP055533681,
- WILSHAW, S. P. et al.: "Production of an acellular amniotic membrane matrix for use in tissue engineering", Tissue Eng., vol. 12, no. 8, August 2006 (2006-08), pages 2117-2129, XP002507640,

## Description

### Scope of the Invention

The present invention relates to forming a collagen matrix or membrane with antimicrobial properties comprising collagen from fetal amniotic membranes, and nanometals also known as metallic nanoparticles according to claim 1.

The present invention allows to forming a product that keeps the biomechanical properties such as flexibility, the capability of being stitchable and low immunogenicity, as it derives from human placenta with the added value of having antimicrobial properties, which makes its use range even wider.

### Background of the Invention

In the last few decades, there has been much development in fighting diseases such as cancer and Alzheimer's disease. In spite of this development in such diseases, obesity and diabetes seem not to be the case as they seem to be worsening instead of improving over time. For the latter, this increase is particularly alarming since its association with other pathologies including diabetic neuropathy, venous or diabetic ulcer, and diabetic foot. All of them represent an enormous amount of public health expenditure, more so considering that there is no cure for hard-to-heal diabetic ulcers.

Today, diabetic ulcers are a serious health problem. Although in the past few years, the treatment for diabetic ulcers has developed considerably, the treatment for chronic diabetic ulcers remains a significant issue with very limited options for treatment. There are multiple treatments for wounds that are innovative, which not always deliver conclusive results. This goes to show that more research is necessary in order to provide proof to change the routine care protocols that are currently used in hospitals. This is crucial considering that patients with chronic diabetic ulcers have to deal with pain, infection, hospitalization and amputations. This means, on one hand, poor quality of life for the patient and additional, considerable health expenditure for the community. This expenditure could be reduced with an appropriate treatment for these chronic ulcers.

In the pursuit of solutions for these complications, various strategies have been explored. Particularly biological preparations, especially products derived from placental tissue, have been the focus of interest of multiple scientific publications in the last 10 years. This is how the use of biological preparations of human origin has proven to be a powerful, secure and effective tool for the treatment of diseases or conditions associated to chronic diseases. This interest is mainly due to their favoring a tissue healing and regeneration process.

Wounds are defined as lesions that cause loss of soft tissue integrity. The healing of a wound, on the other hand, is a biologically and biochemically complex procedure involving the activation of several cell signaling pathways that are commanded by the so called growth factors, whose ultimate goal is tissue regeneration. In order to aid the healing process, the concept of wound dressing is born. Wound dressing is defined as a technique that promotes healing of any wound until remission is achieved. In spite of the existence of wound dressing techniques, there still are wounds for which the wound dressing techniques fail to attain the expected results.

Currently, there are two main ways to perform a wound dressing: the traditional one and the advanced one. The first one is characterized in that it is performed in a dry environment, uses passive dressings, topicals such as antiseptics and antimicrobials are applied, and its frequency is daily. Also, it is a process that is not regenerative and fails to work for complex or infected wounds. This group includes multilayer patches, compression systems and vacuum therapy.

The advanced wound dressing, on the other hand, is performed in a humid physiological environment, uses active dressings, avoids the use of topicals as possible, and the frequency will depend on the local conditions of the wound. These processes also include different known innovative treatments such as the use of biomaterials, amniotic membranes (Epifix/Amniograft), and bio-tissues including Dermagraph and the gel matrix with growth factors including Regranex^{®}.

Diverse scientific evidence has shown that the humid environment is the best suited for the healing process. Among the many effects that the humid environment would have, we may single out preventing cellular desiccation, promoting cellular migration, and promoting collagen synthesis and angiogenesis. These biological effects would translate, in turn, into clinical effects such as reduced pain, reduced time and higher quality of healing.

In the past few years, a new concept in the area of wound dressing has come up which relates to regenerative products, including bio-tissues, as developed by tissue bioengineering in which collagen I and fibroblast growth factors are added to a gelatin layer over a plastic plate. These products present disadvantages in that they must be used at the moment and cannot be stored in the long run, and are not useful for infected wounds (Yildirimer and Seifalian 2014) and the amniotic membranes (Fijan, Hashemi et al. 2014), all of them used in advanced wound dressing.

For bio-tissues from amniotic membranes, their effectiveness has been noticed to come basically from the presence of epithelial cells and the high concentration of growth factors (i.e., EGF, KGF, HGF and FGF). However, it is a disadvantage when it comes to escalating the product due to the need of cryopreserving the product for said components to remain intact.

The product REGRANEX^{®} is a healing agent whose dosage form is of the topical gel type that is derived from platelets. It is used for treating ulcers and wounds of the foot, ankle or leg in individuals suffering from diabetes. It has as a function to help in healing the ulcer, which is attained since it is a natural product that replaces the dead skin and other tissues by attracting cells that repair wounds. This product is capable of stimulating the healing of tissues and modulates the inflammation, but it cannot be used on infected wounds. Its main disadvantage lies in that an increase in the mortality rate secondary to malignancy has been noticed in patients treated with 3 or more REGRANEX^{®} Gel tubes in a post-marketing retrospective cohort study. REGRANEX^{®} Gel should only be used when the benefits are expected to outweigh the risks. REGRANEX^{®} Gel should be used with caution in patients with known malignancy.

Amniograft/EpiFix^{®} is a dry amniotic/chorionic membrane dressing made up by multiple layers including a simple layer of epithelial cells and an avascular connective tissue layer. EpiFix^{®} is handled and dried minimally, thus retaining most of the growth factors, cytokines and extracellular matrix proteins present in the amniotic tissue that allow the soft-tissue regeneration. It can be stored at room temperature, stimulates the healing of tissues and modulates the inflammation, but it cannot be used on infected wounds.

The state of the art includes different ways to heal ulcerous wounds, including both traditional and advanced wound dressing. However, procedures that are completely effective and easy to apply, and that allow for complete recovery of the ulcerated site have not been found. Therefore, there is the need for a product that is easy-to-apply and allows for a quick recovery of the ulcerated site, even though when the wounds are infected.

Document WO 2008/057162 A2 discloses compositions comprising human placental telopeptide collagen, methods of preparing the compositions, methods of their use and kits comprising the compositions.

Document WO 2006/135843 A2 discloses compositions comprising human placental collagen, methods of preparing the compositions, methods of their use and kits comprising the compositions.

Document US 2004/048796 A1 discloses collagenous membranes produced from amnion, herein referred to as a collagen biofabric. The collagen biofabric has the structural integrity of the native non-treated amniotic membrane, i.e., the native tertiary and quaternary structure. Document US 2004/048796 A1 also provides a method for preparing a collagen biofabric from a placental membrane, preferably a human placental membrane having a chorionic and amniotic membrane, by decellularizing the amniotic membrane.

### Summary of the Invention

The invention refers to a manufacturing procedure for a cell- and protein-factor-free collagen matrix or membrane with antimicrobial properties according to claim 1.

The present invention corresponds to a product according to claim 4 that keeps the biomechanical properties such as flexibility, the capability of being stitchable and low immunogenicity, as it derives from human placenta with the added value of having antimicrobial properties, which makes its use range even wider.

The present invention corresponds to a placenta-derived collagen matrix according to claim 4 that is cell- and protein-factor-free, which makes product escalation easier and the placental derivatives not to lose their regenerative capability due to the presence of collagen, main component of the extracellular matrix and responsible for allowing cell migration, which is essential for the cell migration and wound closure process.

Particularly, the invention corresponds to a collagen matrix or membrane with antimicrobial properties according to claim 4 and to the procedure to form said collagen matrix or membrane that comprises collagen from fetal amniotic membranes, and nanometals also known as metallic nanoparticles, according to claim 1.

### Brief Description of the Drawings

The invention will be described below with reference to appended drawings, in which:
Figure 1 shows the graph summarizing the results for an average patient treated with the collagen matrix or membrane with antimicrobial properties of the present invention.
Figure 2 shows results from an antibiotic sensitivity trial performed on the matrix or membrane of the present invention.
Figures 3-4 show the results from antibiotic sensitivity trials performed on a collagen matrix or membrane with antimicrobial properties of the present invention.
Figure 5 shows the sequence of Gram-negative bacteria from genus *Proteus.*
Figure 6 shows the sequence of Gram-positive bacteria from genus *Staphylococcus.*

### Detailed Description of the Invention

The present invention corresponds to a collagen matrix or membrane with antimicrobial properties, comprising collagen from fetal amniotic membranes and metallic nanoparticles.

The present invention also corresponds to the procedure to form said collagen matrix or membrane with antimicrobial properties.

Considering that the base raw material to form the collagen matrix or membrane of the present invention is collagen derived from fetal amniotic membranes, first the legal procedure as set forth for obtaining this type of raw materials will be described.

In this context, the legal statute that is applicable to the donation of placenta, also containing the amniotic membrane, under the Article 153 from Book IX of the Local Health Code, and the Article 16 of its Regulations, states that the consent from the mother for any placenta donation is not necessary. However, following international good practices, the fetal amniotic membranes will be obtained through informed consent by the donating mother as a necessary condition to form a collagen matrix o membrane. In order to use the fetal amniotic membranes, the authorization from the Director of the facilities where the placenta is to be obtained, will be required. An agreement with clinic solves this issue. Regarding the commercial use, the commercialization of products derived from organs and tissues is not expressly forbidden under the Article 153 from Book IX of the Local Health Code or Article 16 of its Regulations. On the particulars, while its direct sale could be debatable, for example, the placenta (since this can be acquired for free and its utilization should be done in the same way), it is not the same with the manufacturing procedure by which the placenta is turned into a therapeutic product (such as the collagen matrix or membrane). On the other hand, in the USA, which is one of the markets where the collagen matrix or membrane of the present invention is intended to be sold, the legal procedure falls under the FDA Standard 361 (Human Cells and Tissue-Based Products). The most important characteristics of this Standard include clinical trials for the product according to the terms by the FDA.

Procedure to form the fetal amniotic membranes:
1.- The donation of the Placenta, which also contains the fetal amniotic membrane, is requested by informed consent. If patient agrees, signature is requested.
2.- At the same time, a blood sample is taken to perform the following serological testing: HIV, Rapid Plasma Reagin (RPR), Hepatitis B Virus Surface Antigen; Hepatitis C Virus Serology, Chagas disease (endemic in Chile), VDRL, and it is sent to a Clinical Laboratory. These results take 7 working days to be available. This is done in order to meet the FDA Standard 361 (Human Cells and Tissue-Based Products).
3.- The placenta then is carried inside a portable cooler, previously disinfected with ethanol, 70 % v/v, and inside a previously-sterilized hermetic container to the laboratory facilities where the tissue is secured. It is worth mentioning at this step that fetal amniotic membranes only from C-section births will be used. This as a means to avoid contamination of the placenta and the fetal amniotic membrane with microorganisms from the urogenital tract of the mother when passing through the birth canal during the regular birth process.
4.- The chorion is separated from the rest of the placenta with the aid of surgical scissors and clamps in order to get the fetal amniotic membrane. This fetal amniotic membrane is washed as many times as necessary in order to remove any rest of blood and clots. Ethanol 70 % v/v is applied if necessary in order to remove any rest of clots.

The manufacturing procedure for the collagen matrix or membrane with antimicrobial properties comprises the following stages:
i) forming collagen from fetal amniotic membranes;
ii) adding and incorporating metallic nanoparticles to the thus formed collagen in order to form the collagen matrix or membrane with antimicrobial properties; and
iii) dehydrating and irradiation of the collagen matrix or membrane with antimicrobial properties in order to form a ready-to-pack product.

### i) Forming collagen from fetal amniotic membranes:

Forming collagen starts on the fetal amniotic membranes. To that end, the tissue corresponding to the fetal amniotic membrane is treated chemically in order to remove any protein rest that might remain therein. This first treatment comprises dipping the tissue in a 0.01 % sodium dodecyl sulfate (SDS) solution for one hour at room temperature. This procedure is performed in agitation by using an orbital shaker and inside a tightly closed container in order to avoid contamination with opportunistic microorganisms existing in the environment.

When this procedure is finished, a second chemical treatment is applied in order to remove any cell that might remain attached to the tissue. To this end, the tissue is dipped in a pH marker-free Trypsin-EDTA solution (colorless) for 1 hour at 37 °C. Three washes are then performed with physiological saline for 15 minutes, each one to remove any cell that might have detached from the tissue during the trypsin wash process. This is also performed in agitation by using an orbital shaker and inside a closed container.

Then, a second treatment is performed with 0.01 % SDS for one hour at room temperature. This procedure is also performed in agitation by using an orbital shaker and inside a closed container. This procedure is performed in order to remove any protein components and cell rests that might have remained after the Trypsin-EDTA treatment. Five washes are then performed with sterile physiological saline, and thus collagen, which is the base raw material of the present invention, is formed.

The collagen formed by this procedure keeps its structural integrity. A decellularization procedure is thus performed on the fetal amniotic membrane. This allows to obtain a product with numerous applications in the field of medicine, such as tendon repair, blood vessels, eye surgeries and wound dressings (Wilshaw SP, Kearney JN, Fisher J, Ingham E. 2006, "Production of an acellular amniotic membrane matrix for use in tissue engineering". Tissue Eng. 2006 Aug;12(8):2117-29) and (Letendre S, LaPorta G, O'Donnell E, Dempsey J, Leonard K. 2009. "Pilot trial of biovance collagen-based wound covering for diabetic ulcers". Adv Skin Wound Care. 2009 Apr; 22(4):161-6).

Then, the collagen thus formed is mounted on an inert support, e.g. a previously sterilized clear plastic mica, to be dehydrated and irradiated and stored in vacuum-closed sleeves at a temperature of -80 °C, pending the serological testing results.

If the serological testing is negative, the second part of the production procedure for the collagen matrix or membrane with antimicrobial properties is carried out. If some test is positive, said matrices are removed, and the procedure is restarted from stage i).

### ii) Adding and incorporating metallic nanoparticles to the thus formed collagen:

In an embodiment of the present invention, an aqueous solution is prepared in sterile and filtered water, comprising metallic nanoparticles of:
a) copper or
b) copper/silver.

In a further embodiment of the present invention, an aqueous solution is prepared comprising a Copper/Silver/Gold mixture. In this case, the solvent to be used is citrate.

When preparing the copper (Cu) nanoparticle aqueous solution, copper nanoparticles of a size ranging from 25 to 60 nm are used, and the solution remains at a final copper concentration of 1 mg/mL.

As for preparing the copper/silver aqueous solution, the same size of copper particles as above is used, and the silver (Ag) nanoparticle size is 100 nm or less. On the other hand, the copper nanoparticle concentration is kept at 1 mg/mL in the final solution, while for the silver nanoparticle concentration is 2.5-5 ng/mL. For both nanoparticles, purity is ≥ 99.5 %.

In the case of adding quantumdots (qdts), a (cadmium) quantumdot solution at a concentration of 0.1-10 nM is used.

When using a Copper/Silver/Gold solution, the nanoparticle dissolution is carried out in a citrate solution. The purity, size and concentration of the copper and silver nanoparticles is kept exactly the same as above. The size of the gold nanoparticles is 5 nm, and the concentration of the gold nanoparticles in the final solution is 5.5E+13 particles per mL

Adding nanoparticles is carried out by embedding the collagen formed in the aqueous solution containing the metallic nanoparticles, and it is placed in an electrophoresis chamber and submitted to an electric field of 50 Volts for 30 min for the nanoparticles to incorporate into the formed collagen and spread evenly. This way, a collagen matrix or membrane is formed.

### iii) Dehydrating and irradiating the collagen matrix or membrane with antimicrobial properties in order to form a ready-to-pack product:

The collagen matrix or membrane with antimicrobial properties is dehydrated and is irradiated for 30 min with UV light in order to obtain the product that is ready for its final packing. Dehydration is carried out slowly in a biosafety cabinet and under conditions of sterile circulating air (HEPA Filter 99.95 %).

The formed product, having copper, copper/silver or qdt nanoparticles added, is the collagen matrix or membrane with antimicrobial properties, which can be lyophilized and subsequently dissolved in an organic solvent or put in a container with atomizer in order to generate a product in the form of an easy-to-apply spray that can be sold in drug stores.

The collagen matrix or membrane with antimicrobial properties presents regenerative properties and broad antimicrobial activity spectrum when copper, copper/silver or copper/silver/gold nanoparticles are added. In the case of adding a qdt mixture, a product with regenerative properties and reduced antimicrobial activity spectrum designed for some Gram (-) and Gram (+) S. *aureus* is formed.

### Results obtained with the collagen matrix or membrane with antimicrobial activity

### 1.- Tissue Regeneration.

Once formed, the collagen matrix or membrane with antimicrobial activity is stored under tight-seal, room-temperature conditions. When used in the patient, it is reconstituted with physiological saline (0.9 % NaCI).

Once reconstituted, it is placed on the wound to be treated, and the patient is checked every 7 days. A new patch with the collagen matrix or membrane with antimicrobial activity is placed on the treated site every 7 days. The site is then isolated from the environment by incorporating a traditional dressing (gauze).

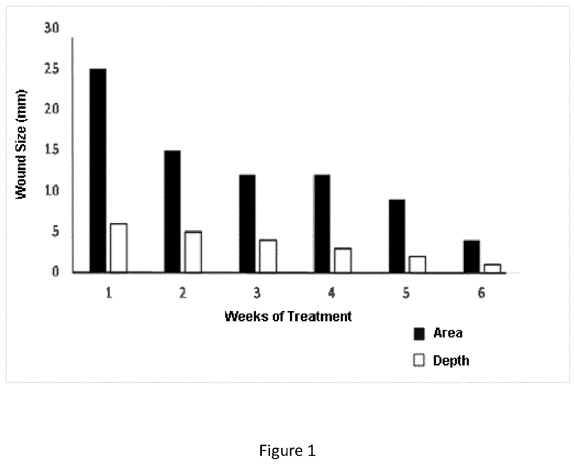

After 5-6 applications, a reduction of the area and depth of the wound in an average of 80 % is attained. Figure 1 shows these results.

Figure 1 shows the graph summarizing the results for an average patient treated with a collagen matrix or membrane with antimicrobial activity after 6 product applications. The bars in black show the data for the wound area, and the bars in white are data for the wound depth in millimeters (mm). As noticed in Figure 1, the wound area is substantially reduced as the weeks of treatment with the product of the present invention went by, a decrease of 80 % of the wound area being thus attained. The same happens with wound depth, where a decrease of 80 % of the wound depth is also attained.

### 2.- Microbicide power of the collagen matrix or membrane with antimicrobial activity.

In order to assert the antimicrobial capability of the collagen matrix or membrane, lab testing was carried out. Patient biopsies were collected from different types of infected wounds (neuropathic diabetic foot and biofilms). These samples were homogenized and set to culture for 24 hours to observe if there was bacterial growth or not. When bacterial growth is observed, colonies of these cultures were isolated, and with them, a test for microbial sensitivity to the action of nanometals was carried out. It is noteworthy that they were collected from Gram-negative and -positive bacteria biopsies, and both were tested for this sensitivity trial.

In order for this trial to be performed, antibiotics commonly used for eliminating these bacteria and at set concentrations were used as controls. Particularly in this case, the antibiotics used as controls were as follows: Erythromycin (15 mg), Clarithromycin (CLR) (15 mg), Penicillin (10 mg) Ampicillin (AM) (10 mg), Ciprofloxacin (CIP) (5 mg), Vancomycin (30 mg).

In order to analyze the effectiveness of copper nanometals and its effectiveness as antimicrobial agents, three concentrations, 1, 5 and 10 mg/ml, were used. To show that the nanometal particles that are present in the collagen matrix or membrane effectively have antimicrobial activity, as control for this trial, a copper-nanometal-particle-free collagen matrix or membrane was used.

The trial was carried out as follows: On the surface of an agar Müller-Hinton plate, 100 ul of a bacterial solution (OD600: 0,4) were inoculated and seeded evenly for bacterial colonies to grow. Then, filter paper discs soaked with known concentrations of the different antibiotics are placed. The antibiotic will spread from the agar filter paper radiallly. The plate was incubated for 18-24 hours at 37 °C, and then the growth inhibition halos are measured by interpreting them according to the available data in databases. The results are expressed as: Sensitive (S), Intermediate or Moderately sensitive (I) and Resistant (R). Table 1 shows them.

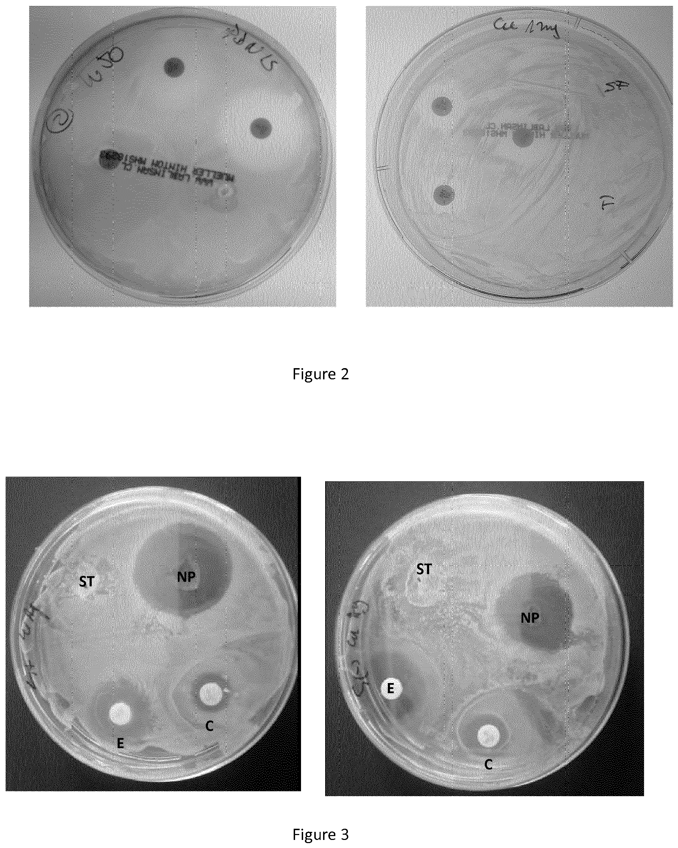

For both Gram-negative bacteria and Gram-positive bacteria, a copper concentration of 1 mg/mL was enough to inhibit the bacterial growth. The copper-free collagen matrix or membrane, on the other hand, was not capable to inhibit the growth. Figure 2 shows the results obtained for this trial. Figure 2 shows the results for the antibiotic sensitivity trial. The halos surrounding the discs indicate bacterial growth inhibition. The image to the left shows a Gram-negative bacteria trial. The image to the right shows a result for Gram-positive bacteria. F: nanometal-free collagen matrix or membrane (absence of copper nanoparticles) SF: collagen matrix or membrane with nanometals at a concentration of 1 mg/mL. CIP: Ciprofloxacin, AM: Ampicillin. CLR: Clarithromycin.

### Table 1: Antibiotic sensitivity

**Table 1: Quantification of inhibition halos obtained in antibiotic sensitivity trials.**

| **Antibiotic** | **Plate 1 (Gram +) (mm)** | **Sensitivity Level** | **Plate 2 (Gram +) (mm)** | **Sensitivity Level** |
|---|---|---|---|---|
| | | | | |
| CIP | 28 | Sensitive | 19 | Sensitive |
| AM | 0 | Resistant | 12 | Intermediate |
| CLR | 15 | Intermediate | 18 | Sensitive |
| Copper- | 7 | Intermediate | 10 | Sensitive |
| containing Matrix | | | | |
| **Copper-free Matrix** | **0** | **0** | **N/A** | |

To determine the inhibition halo, the diameter surrounding the sense disc on which bacterial growth was not observed was measured on the plate seeded with bacteria in the presence of different antibiotics. In this case, the inhibition halo is expressed in mm. According to the halo size, the bacteria are classified as sensitive to the antibiotic action, resistant to the antibiotic action or of intermediate resistance to the antibiotic action (data that is published in databases). The table data named Plate 1 is the data obtained for a typical trial as shown in Figure 2 left (Gram-negative bacteria). The data on table Plate 2 is the data obtained for a typical trial as shown in Figure 2 right (Gram-positive bacteria). The following antibiotics were studied in this trial: CIP: Ciprofloxacin, AM: Ampicillin. CLR: Clarithromycin. The values shown represent the average for three experiments. Additionally, the purpose of this trial is to rule out that the matrix alone presents some level of antimicrobial action, therefore, a copper-free matrix was used as control. In that case, the sensitivity level was classified as N/A (Not Applicable).

Gram-negative bacteria belong to genus *Proteus,* and Gram-positive bacteria belong to genus *Staphylococcus.*

### 3.- Uses of the collagen matrix or membrane and its derivatives:

The use of the collagen matrix or membrane and its derivative products is recommended for wounds of diabetic foot, venous ulcers, sores, burns, epidermolysis bullosa (glass foot) and wounds caused by chafing with orthopedic appliances, according to claim 11.

Additionally, since this is a product that is reabsorbed by the skin and extremely thin, its use can be supplemented with existing therapies and treatments, such as hydrocolloids that allow to hydrating the treated skin, alginates that allow to absorbing secretions and/or activated carbon to neutralize odors caused by such wounds.

### Results with Cu/Ag and Cu/Ag/Au

In order to assert the antimicrobial capability of the collagen matrix or membrane, lab testing was carried out. Patient biopsies were collected from different types of infected wounds (neuropathic diabetic foot and biofilms). These samples were homogenized and set to culture for 24 hours to observe if there was bacterial growth or not. When bacterial growth was observed, colonies of these cultures were isolated, and with them, a test for microbial sensitivity to the action of nanometals was carried out. In this case, Cu/Ag solutions and Cu/Ag/Au solutions were tested. It is noteworthy that they were collected from Gram-negative and -positive bacteria biopsies, and both were tested for this sensitivity trial. The trial was carried out as follows: On the surface of an agar Müller-Hinton plate, 100 ul of a bacterial solution (OD600: 0.4) were inoculated and seeded evenly for bacterial colonies to grow. Then, filter paper discs soaked with known concentrations of the different antibiotics are placed. Particularly in this case, Ciprofloxacin (5 mg) and Erythromycin (15 mg) were used as controls. Additionally for each case, a collagen matrix that was not treated with metallic nanoparticles was also used.

Figure 3 shows the results for the antibiotic sensitivity trial for a Gram-positive bacterium (left) and a Gram-negative bacterium (right) isolated from the infected wound in patients with diabetic foot. Figure 3 represents the results obtained for a collagen matrix or membrane with Cu/Ag nanoparticles and free of metallic nanoparticles.

As Figure 3 shows, the halos surrounding the discs indicate inhibition of the bacterial growth. The image to the left shows a result for some Gram-positive bacteria. The image to the right shows a Gram-negative bacteria trial. In this case, both bacteria were isolated from infected wounds of patients with diabetic foot. The symbols in Figure 3 are as follows: ST: nanometal-free filtered screen (absence of copper and silver nanoparticles). NP: filtered membrane with copper, at a concentration of 1 mg/ml, and silver, 5 ng/mL, nanometals. C: Ciprofloxacin, E: Erythromycin.

The same metallic nanoparticle sensitivity experiment above was carried out in the presence of a mixture of copper, silver and gold nanoparticles. Again bacteria were seeded evenly for bacterial colonies to grow. Then, filter paper discs soaked with known concentrations of the different antibiotics are placed. As in the former case, Ciprofloxacin (5 mg) and Erythromycin (15 mg) were used as controls, and a collagen matrix or membrane that was not treated with metallic nanoparticles was also used.

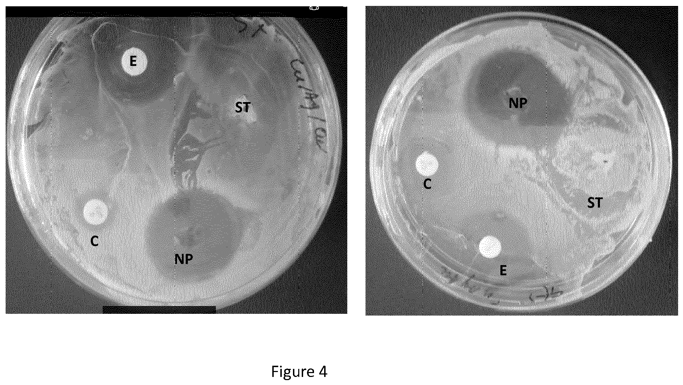

Figure 4 shows the results for the antibiotic sensitivity trial for a Gram-positive bacterium (left) and a Gram-negative bacterium (right) isolated from the infected wound in patients with diabetic foot. Figure 4 represents the results obtained for a collagen matrix or membrane with Cu/Ag/Au nanoparticles and free of metallic nanoparticles.

As Figure 4 shows, the halos surrounding the discs indicate inhibition of the bacterial growth. The image to the left shows a result for some Gram-positive bacteria. The image to the right shows a Gram-negative bacteria trial. In this case, both bacteria were isolated from infected wounds of patients with diabetic foot. The symbols in Figure 4 are as follows: ST: nanometal-free filtered screen (absence of copper and silver nanoparticles). NP: filtered membrane with copper, at a concentration of 1 mg/ml, silver, 5 ng/mL, and gold, at 5,5E+13 particles per mL, nanometals. C: Ciprofloxacin, E: Erythromycin.

Additionally, DNA was extracted from these bacteria, and a sequencing was carried out in order to identify the treated bacteria. Once obtained, the sequence is uploaded to a US NIH database and is compared with sequences existing in the database.

As a result of the comparison, the Gram-negative bacteria sequence belonged to genus *Proteus,* and the Gram-positive bacteria sequence, to genus *Staphylococcus.*

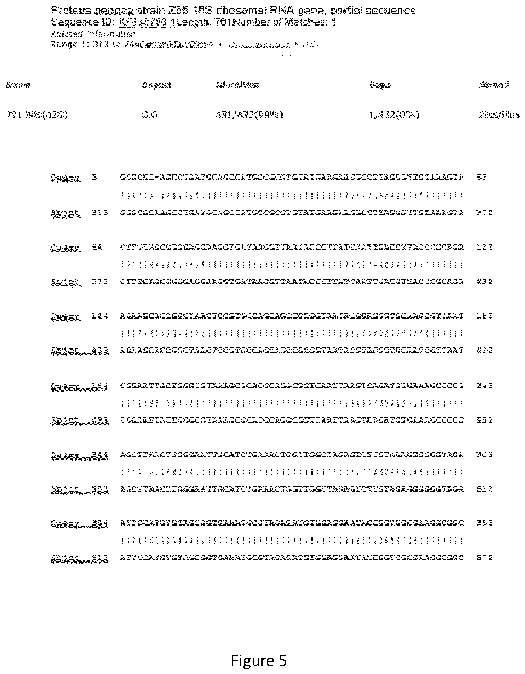

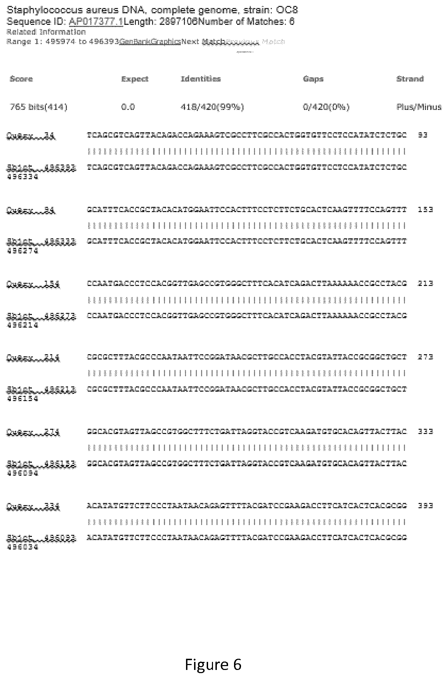

Figures 5 and 6, respectively, show the results obtained for the Gram-negative and Gram-positive bacteria after the sequence comparison. Figure 5 shows the Gram-negative bacteria sequence belonging to a patient's isolate of genus *Proteus,* and Figure 6 shows the Gram-positive bacteria sequence belonging to a patient's isolate of genus *Staphylococcus.*

## Claims

1. Manufacturing procedure for a cell- and protein-factor-free collagen matrix or membrane with antimicrobial properties, **CHARACTERIZED in that** it consists of the following steps:
i) forming collagen from fetal amniotic membranes;
ii) adding and incorporating metallic nanoparticles to the thus formed collagen in order to form the collagen matrix or membrane with antimicrobial properties; and
iii) dehydrating and irradiating the collagen matrix or membrane with antimicrobial properties in order to form a ready-to-pack product;
and **in that**, for the collagen to be formed from the fetal amniotic membrane, this is treated chemically in order to remove any protein rest that might remain therein in a first treatment comprising dipping the fetal amniotic membrane in a 0.01 % sodium dodecyl sulfate (SDS) solution for one hour at room temperature, in agitation by using an orbital shaker and inside a tightly closed container, and
when the first treatment is finished, a second chemical treatment is performed in order to remove any cell that might remain attached to the fetal amniotic membrane, for which the fetal amniotic membrane is dipped in a pH marker-free Trypsin-EDTA solution (colorless) for 1 hour at 37 °C; then, three washes are then performed with physiological saline for 15 minutes, each one to remove any cell that might have detached from the tissue during the trypsin wash process, and
when the second treatment is finished, a new chemical treatment is performed with 0.01 % SDS for one hour at room temperature, in agitation by using an orbital shaker and inside a closed container, for the collagen to finally form from amniotic membrane, and
**in that** for metallic nanoparticles to be added and incorporated into the formed collagen, the collagen formed is embedded in an aqueous solution containing the metallic nanoparticles, and it is placed in an electrophoresis chamber and submitted to an electric field of 50 Volts for 30 min for the nanoparticles to be incorporated into the formed collagen and spread evenly.

2. Manufacturing procedure for a cell- and protein-factor-free collagen matrix or membrane with antimicrobial properties according to Claim 1, **CHARACTERIZED in that**, to dehydrate the collagen matrix or membrane, dehydration is carried out slowly in a biosafety cabinet and under conditions of sterile circulating air (HEPA Filter 99.95 %).

3. Manufacturing procedure for a cell- and protein-factor-free collagen matrix or membrane with antimicrobial properties according to Claim 2, **CHARACTERIZED in that** the collagen matrix or membrane with antimicrobial properties is irradiated for 30 min with UV light.

4. Cell- and protein-factor-free collagen matrix or membrane with antimicrobial properties, obtained by the manufacturing procedure according to claims 1 to 3.

5. Cell- and protein-factor-free collagen matrix or membrane according to Claim 4, **CHARACTERIZED in that** the metallic nanoparticles are copper nanoparticles contained in an aqueous solution, wherein the copper nanoparticles have a particle size of 25-60 nm, and wherein the solution has a final copper concentration of 1 mg/mL.

6. Cell- and protein-factor-free collagen matrix or membrane according to Claim 4, **CHARACTERIZED in that** the metallic nanoparticles are a mixture of copper and silver contained in an aqueous solution, wherein the copper nanoparticles have a particle size of 25-60 nm, and the silver nanoparticles have a particle size of 100 nm or less.

7. Cell- and protein-factor-free collagen matrix or membrane according to Claim 6, **CHARACTERIZED in that** the solution has a final copper concentration of 1 mg/mL and a silver nanoparticle concentration of 2.5-5 ng/mL.

8. Cell- and protein-factor-free collagen matrix or membrane according to Claim 4, **CHARACTERIZED in that** the metallic nanoparticles are a mixture of cadmium quantumdots contained in an aqueous solution at a concentration of 0.1-10 nM.

9. Cell- and protein-factor-free collagen matrix or membrane according to Claim 4, **CHARACTERIZED in that** the metallic nanoparticles are a mixture of copper, silver and gold contained in an aqueous solution, wherein the copper nanoparticles have a particle size of 25-60 nm, the silver nanoparticles have a particle size of 100 nm or less, and the gold nanoparticles have a particle size of 5 nm.

10. Cell- and protein-factor-free collagen matrix or membrane according to Claim 9, **CHARACTERIZED in that** the solution has a final copper concentration of 1 mg/mL and a silver nanoparticle concentration of 2.5-5 ng/mL, and a concentration of the gold nanoparticles in the final solution of 5.5E+13 particles per mL.

11. Cell- and protein-factor-free collagen matrix or membrane with antimicrobial properties according to Claims 4-10, for use in treating diabetic neuropathies, venous or diabetic ulcers and diabetic foot.

12. Cell- and protein-factor-free collagen matrix or membrane with antimicrobial properties according to Claims 4-10, for use in treating sores, burns, epidermolysis bullosa (glass foot) and wounds caused by chafing with orthopedic appliances.

13. Cell- and protein-factor-free collagen matrix or membrane with antimicrobial properties according to Claims 4-10, for use in treating Gram-negative and Gram-positive bacterial wound infections.

## Patentansprüche

1. Herstellungsverfahren für eine zell- und proteinfaktorfreie Kollagenmatrix oder -membran mit antimikrobiellen Eigenschaften, **dadurch gekennzeichnet, dass** es aus den folgenden Schritten besteht:
i) Bilden von Kollagen aus fötalen Amnionmembranen;
ii) Hinzufügen und Einbauen von metallischen Nanopartikeln in das so gebildete Kollagen, um die Kollagenmatrix oder -membran mit antimikrobiellen Eigenschaften zu bilden; und
iii) Dehydrieren und Bestrahlen der Kollagenmatrix oder -membran mit antimikrobiellen Eigenschaften, um ein verpackungsfertiges Produkt zu bilden;
und dadurch dass, für das Bilden des Kollagens aus der fötalen Amnionmembran diese chemisch behandelt wird, um jegliche darin möglicherweise verbliebene Proteinreste in einer ersten Behandlung zu entfernen, die das Eintauchen der fötalen Amnionmembran in eine 0,01 %-ige Natriumdodecylsulfatlösung (SDS) für eine Stunde bei Raumtemperatur unter Rühren mit Hilfe eines Kreisschüttlers und in einem dicht verschlossenen Behälter umfasst, und
nach Beendigung der ersten Behandlung eine zweite chemische Behandlung durchgeführt wird, um jegliche an der fötalen Amnionmembran möglicherweise haften gebliebene Zellen zu entfernen, wofür die fötale Amnionmembran eine Stunde lang bei 37°C in eine pH-Marker-freie Trypsin-EDTA-Lösung (farblos) getaucht wird; anschließend, drei 15-minütige Waschvorgänge mit physiologischer Kochsalzlösung durchgeführt werden, um jegliche Zellen zu entfernen, die sich während des Trypsin-Waschvorgangs von dem Gewebe gelöst haben könnten, und
nach Beendigung der zweiten Behandlung, eine neue chemische Behandlung mit 0,01 %-iger SDS für eine Stunde bei Raumtemperatur unter Rühren mit einem Kreisschüttler und in einem geschlossenen Behälter durchgeführt wird, damit sich das Kollagen schließlich aus der Amnionmembran bildet, und
dadurch dass für die Zugabe und den Einbau von metallischen Nanopartikeln in das gebildete Kollagen, das gebildete Kollagen in eine wässrige Lösung eingelegt wird, die die metallischen Nanopartikel enthält, und es in eine Elektrophoresekammer gelegt und 30 Minuten lang einem elektrischen Feld von 50 Volt ausgesetzt wird, damit die Nanopartikel in das gebildete Kollagen eingebaut werden und sich gleichmäßig verteilen.

2. Herstellungsverfahren für eine zell- und proteinfaktorfreie Kollagenmatrix oder -membran mit antimikrobiellen Eigenschaften nach Anspruch 1, **dadurch gekennzeichnet, dass** zur Dehydrierung der Kollagenmatrix oder -membran, die Dehydrierung langsam in einer Biosicherheitskabine und unter sterilen Umluftbedingungen (HEPA-Filter 99,95 %) durchgeführt wird.

3. Herstellungsverfahren für eine zell- und proteinfaktorfreie Kollagenmatrix oder -membran mit antimikrobiellen Eigenschaften nach Anspruch 2, **dadurch gekennzeichnet, dass** die Kollagenmatrix oder -membran mit antimikrobiellen Eigenschaften 30 Minuten lang mit UV-Licht bestrahlt wird.

4. Zell- und proteinfaktorfreie Kollagenmatrix oder -membran mit antimikrobiellen Eigenschaften, erhalten durch das Herstellungsverfahren nach einem der Ansprüche 1 bis 3.

5. Zell- und proteinfaktorfreie Kollagenmatrix oder -membran nach Anspruch 4, **dadurch gekennzeichnet, dass** es sich bei den metallischen Nanopartikeln um Kupfer-Nanopartikel handelt, die in einer wässrigen Lösung enthalten sind, wobei die Kupfer-Nanopartikel eine Partikelgröße von 25-60 nm haben, und wobei die Lösung eine Kupfer-Endkonzentration von 1 mg/ml aufweist.

6. Zell- und proteinfaktorfreie Kollagenmatrix oder -membran nach Anspruch 4, **dadurch gekennzeichnet, dass** die metallischen Nanopartikel ein Gemisch aus Kupfer und Silber sind, das in einer wässrigen Lösung enthalten ist, wobei die Kupfer-Nanopartikel eine Teilchengröße von 25-60 nm haben und die Silber-Nanopartikel eine Teilchengröße von 100 nm oder weniger haben.

7. Zell- und proteinfaktorfreie Kollagenmatrix oder -membran nach Anspruch 6, **dadurch gekennzeichnet, dass** die Lösung eine Kupfer-Endkonzentration von 1 mg/ml und eine Silber-Nanopartikelkonzentration von 2,5-5 ng/ml aufweist.

8. Zell- und proteinfaktorfreie Kollagenmatrix oder -membran nach Anspruch 4, **dadurch gekennzeichnet, dass** die metallischen Nanopartikel ein Gemisch aus Cadmium-Quantenpunkten sind, die in einer wässrigen Lösung in einer Konzentration von 0,1-10 nM enthalten sind.

9. Zell- und proteinfaktorfreie Kollagenmatrix oder -membran nach Anspruch 4, **dadurch gekennzeichnet, dass** die metallischen Nanopartikel ein Gemisch aus Kupfer, Silber und Gold sind, das in einer wässrigen Lösung enthalten ist, wobei die Kupfer-Nanopartikel eine Teilchengröße von 25-60 nm haben, die Silber-Nanopartikel eine Teilchengröße von 100 nm oder weniger haben und die Gold-Nanopartikel eine Teilchengröße von 5 nm haben.

10. Zell- und proteinfaktorfreie Kollagenmatrix oder -membran nach Anspruch 9, **dadurch gekennzeichnet, dass** die Lösung eine Kupfer-Endkonzentration von 1 mg/ml und eine Silber-Nanopartikelkonzentration von 2,5-5 ng/ml, sowie eine Gold-Nanopartikelkonzentration in der Endlösung von 5,5E+13 Partikeln pro ml aufweist.

11. Zell- und proteinfaktorfreie Kollagenmatrix oder -membran mit antimikrobiellen Eigenschaften nach den Ansprüchen 4-10 zur Verwendung bei der Behandlung von diabetischen Neuropathien, venösen oder diabetischen Geschwüren und diabetischem Fuß.

12. Zell- und proteinfaktorfreie Kollagenmatrix oder -membran mit antimikrobiellen Eigenschaften nach den Ansprüchen 4-10 zur Verwendung bei der Behandlung von Wunden, Verbrennungen, Epidermolysis bullosa (Glasfuß) und Verletzungen, die durch Scheuern mit orthopädischen Geräten verursacht werden.

13. Zell- und proteinfaktorfreie Kollagenmatrix oder -membran mit antimikrobiellen Eigenschaften gemäß den Ansprüchen 4-10 zur Verwendung bei der Behandlung von gramnegativen und grampositiven bakteriellen Wundinfektionen.

## Revendications

1. Procédure de production d'une matrice ou d'une membrane de collagène exempte de cellules et de facteurs-protéines et présentant des propriétés antimicrobiennes, **caractérisée en ce qu'**elle consiste des étapes suivantes :
i) former du collagène à partir de membranes amniotiques foetales ;
ii) ajouter et incorporer des nanoparticules métalliques au collagène ainsi formé afin de former la matrice ou la membrane de collagène présentant des propriétés antimicrobiennes ; et
iii) déshydrater et irradier la matrice ou la membrane de collagène présentant des propriétés antimicrobiennes afin de former un produit prêt pour l'emballage;
et **en ce que**, pour que le collagène se forme à partir de la membrane amniotique fœtale, celle-ci est traitée chimiquement afin d'éliminer toute protéine résiduelle qu'elle pourrait encore contenir avec un premier traitement comprenant l'immersion de la membrane amniotique fœtale dans une solution à 0,01 % de dodécylsulfate de sodium (SDS) pendant une heure à la température ambiante, sous agitation, en utilisant un agitateur orbital et à l'intérieur d'un conteneur fermé hermétiquement, et
lorsque le premier traitement est terminé, un second traitement chimique est réalisé afin d'éliminer toute cellule qui pourrait rester attachée à la membrane amniotique fœtale, pour lequel la membrane amniotique fœtale est plongée dans une solution de trypsine-EDTA (incolore) sans marqueur de pH pendant 1 heure à 37 °C ; ensuite, trois lavages sont réalisés avec une solution physiologique saline pendant 15 minutes, chacun permettant d'éliminer toute cellule qui pourrait avoir été détachée du tissu lors du processus de lavage à la trypsine, et
lorsque le second traitement est terminé, un nouveau traitement chimique est réalisé avec du SDS à 0,01 % pendant une heure à la température ambiante, sous agitation, en utilisant un agitateur orbital et à l'intérieur d'un conteneur fermé hermétiquement, pour que le collagène se forme finalement à partir de la membrane amniotique, et
**en ce que**, pour que des nanoparticules métalliques soient ajoutées et incorporées dans le collagène formé, le collagène formé est incorporé dans une solution aqueuse contenant les nanoparticules métalliques, et est placé dans une chambre d'électrophorèse et soumis à un champ électrique de 50 volts pendant 30 minutes pour que les nanoparticules soient ajoutées dans le collagène formé et réparties uniformément.

2. Procédure de production d'une matrice ou d'une membrane de collagène exempte de cellules et de facteurs-protéines et présentant des propriétés antimicrobiennes selon la revendication 1, **caractérisée en ce que**, pour déshydrater la matrice ou la membrane de collagène, la déshydratation est réalisée lentement dans une enceinte de sécurité biologique et dans des conditions de circulation d'air stérile (filtre HEPA 99,95 %).

3. Procédure de production d'une matrice ou d'une membrane de collagène exempte de cellules et de facteurs-protéines et présentant des propriétés antimicrobiennes selon la revendication 2, **caractérisée en ce que** la matrice ou la membrane de collagène présentant des propriétés antimicrobiennes est irradiée pendant 30 minutes à la lumière UV.

4. Matrice ou membrane de collagène exempte de cellules et de facteurs-protéines et présentant des propriétés antimicrobiennes, obtenue par la procédure de production selon les revendications 1 à 3.

5. Matrice ou membrane de collagène exempte de cellules et de facteurs-protéines selon la revendication 4, **caractérisée en ce que** les nanoparticules métalliques sont des nanoparticules de cuivre contenues dans une solution aqueuse, les nanoparticules de cuivre ayant une taille de particules de 25-60 nm, et la solution présentant une concentration finale en cuivre de 1 mg/mL.

6. Matrice ou membrane de collagène exempte de cellules et de facteurs-protéines selon la revendication 4, **caractérisée en ce que** les nanoparticules métalliques sont un mélange de cuivre et d'argent contenu dans une solution aqueuse, les nanoparticules de cuivre ayant une taille de particules de 25-60 nm et les nanoparticules d'argent ayant une taille de particules inférieure ou égale à 100 nm.

7. Matrice ou membrane de collagène exempte de cellules et de facteurs-protéines selon la revendication 6, **caractérisée en ce que** la solution présente une concentration finale en cuivre de 1 mg/mL et une concentration en nanoparticules d'argent de 2,5-5 ng/mL.

8. Matrice ou membrane de collagène exempte de cellules et de facteurs-protéines selon la revendication 4, **caractérisée en ce que** les nanoparticules métalliques sont un mélange de points quantiques de cadmium contenus dans une solution aqueuse selon une concentration de 0,1-10 nM.

9. Matrice ou membrane de collagène exempte de cellules et de facteurs- protéines selon la revendication 4, **caractérisée en ce que** les nanoparticules métalliques sont un mélange de cuivre, d'argent et d'or contenu dans une solution aqueuse, les nanoparticules de cuivre ayant une taille de particules de 25-60 nm, les nanoparticules d'argent ayant une taille de particules inférieure ou égale à 100 nm et les nanoparticules d'or ayant une taille de particules de 5 nm.

10. Matrice ou membrane de collagène exempte de cellules et de facteurs-protéines selon la revendication 9, **caractérisée en ce que** la solution présente une concentration finale en cuivre de 1 mg/mL et une concentration en nanoparticules d'argent de 2,5-5 ng/mL, et une concentration en particules d'or dans la solution finale de 5,5 E+13 particules par mL.

11. Matrice ou membrane de collagène exempte de cellules et de facteurs-protéines et présentant des propriétés antimicrobiennes selon les revendications 4 à 10, pour une utilisation dans le traitement de neuropathies diabétiques, d'ulcères veineux ou diabétiques et de pied diabétique.

12. Matrice ou membrane de collagène exempte de cellules et de facteurs-protéines et présentant des propriétés antimicrobiennes selon les revendications 4 à 10, pour une utilisation dans le traitement de plaies, de brûlures, de l'épidermolyse bulleuse (pied en verre), et des blessures générées par le frottement de dispositifs orthopédiques.

13. Matrice ou membrane de collagène exempte de cellules et de facteurs-protéines et présentant des propriétés antimicrobiennes selon les revendications 4 à 10, pour une utilisation dans le traitement d'infections de blessures par une bactérie Gram-négative ou Gram-positive.
